# EUROPÄISCHE PATENTSCHRIFT

(11) **EP 4 079 952 B1**
(45) Veröffentlichungstag und Bekanntmachung des Hinweises auf die Patenterteilung: **27.09.2023**
(21) Anmeldenummer: 22020167.7
(22) Anmeldetag: 12.04.2022
(51) Int. Cl.: D04H 1/46, A61F 13/511, D04H 1/4258

(54) **DECKBLATT**
COVER SHEET
FEUILLE DE DESSUS

(30) Priorität: 19.04.2021 DE 202021001414 U
(43) Veröffentlichungstag der Anmeldung: 26.10.2022
(73) Patentinhaber: Sandler AG, 95126 Schwarzenbach/Saale (DE)
(72) Erfinder: Bayer, Ralf, 95213 Münchberg (DE); Bernhuber, Uwe, 95032 Hof (DE); Schuberth, Martin, 95236 Stammbach (DE); Summa, Lorenz, 95126 Schwarzenbach/Saale/OT Martinlamitz (DE)
(74) Vertreter: Grünecker Patent- und Rechtsanwälte PartG mbB

(56) Entgegenhaltungen:
- WO-A1-2014/026207
- WO-A1-2015/041862
- US-A- 5 253 397
- US-A1- 2015 044 926
- US-A1- 2020 138 642

## Beschreibung

Prinzipiell haben persönliche Hygieneprodukte folgenden, schematischen Aufbau:
- Deckblatt als zum Körper hingewandte Lage
- Aufnahme- und Verteillage.
- Speicherlage
- Rückenblatt als flüssigkeitsdichte Außenlage

Das Deckblatt hat dabei die Aufgabe dem Benutzer ein trockenes, weiches, und angenehmes Tragegefühl zu vermitteln, sowie auftreffende Flüssigkeit schnell anzusaugen und an die nachfolgende Aufnahme- und Verteillage weiterzuleiten, sowie die Rücknässung zu minimieren. Die Aufnahme- und Verteillage dient zur raschen Aufnahme der Flüssigkeit aus dem Deckblatt und gewährleistet die Verteilung der Flüssigkeit über die gesamte Fläche eines Hygieneproduktes. Letztendlich wird die Flüssigkeit in der Speicherschicht immobilisiert.

Bisherige Deckblätter für sogenannte Personal Care Produkte (z. B. Damenbinden, Babywindeln und Inkontinenzprodukte) bestehen zum großen Teil aus Chemiefasern.

In der Vergangenheit wurden Deckblätter vornehmlich aus Chemiefasern hergestellt, die aus natürlichen Polymeren aber auch Synthesepolymeren wie Polyester bestehen. Diese Vliesstoffe wurden aus Cellulose-Pulp-Fasern oder aber mittels Kardierung aus Cellulose- oder Polyester-Stapelfasern hergestellt und mittels Bindemitteln verfestigt.

Üblicherweise kommen für die Herstellung von Deckblättern Chemiefasern aus synthetischen, thermoplastischen Polymeren zum Einsatz. Bevorzugt werden dabei Faser oder Filamente aus Polymeren wie Polypropylen, Polyethylen/Polypropylen-Bikofaser oder Polyethylen/Polyester-Bikokomponentenfasern benutzt. Daraus können aus Filamentvliesstoffen wie Spinnvliese aber auch Stapelfaservliesstoffe mittels kardieren hergestellt werden. Vliesstoffe aus derartigen Polymeren weisen im Allgemeinen hydrophobe Eigenschaften auf.

Diese Fasern oder Vliesstoffe werden bei der Herstellung mit einer hydrophilen Beschichtung versehen, sodass das ein so hergestelltes Deckblatt zunächst auftretende Flüssigkeit annimmt und an die Speicherschicht weiterleitet. Die hydrophile Beschichtung wird dabei größtenteils von auftretenden Körperflüssigkeiten, beispielsweise Urin, mit abgewaschen, sodass die hydrophoben Eigenschaften der Polymere wie Polypropylen und Polyethylen zum Tragen kommen. Das Deckblatt weist nach dem Abwaschen der Hydrophilierung durch die Flüssigkeitsbeaufschlagung hydrophobe Eigenschaften und fungiert als Sperrschicht für noch nicht in der Speicherschicht gebundene Flüssigkeit.

Zur Verfestigung werden die thermische Verbindung mittels Heißluft oder Kalandrieren bevorzugt. Mechanische Verbindung mittels Nadelung durch Metallnadeln oder Wasserstrahlen ist auch bekannt.

Für den Tragekomfort eines persönlichen Hygieneprodukts sind die Parameter Ansaugzeit und Rücknässung eines Deckblatts essentiell.

Auf das Deckblatt auftreffende Flüssigkeit muss schnell an die Verteilerlage weitergegeben werden. Messtechnisch wird dies mit dem Parameter Ansaugzeit, gemessen in Sekunden beschrieben. Je geringer die Ansaugzeit ist, umso schneller kann die Flüssigkeit von der Benutzerseite hin zur Verteilerlage und Saugseite transportiert werden. Üblicherweise weist ein handelsübliches Deckblatt eine Ansaugzeit von kleiner 5 Sekunden, bevorzugt 3 Sekunden auf.

Überschüssige Flüssigkeit, die noch nicht in der Speicherlage gebunden ist, darf nicht auf die Benutzerseite zurückfließen. Dieser Effekt wird mittels dem Parameter Rücknässung messtechnisch erfasst und in "Gramm" ausgedrückt. Üblicherweise weist ein handelsübliches Deckblatt eine Rücknässung von kleiner 0,40 Gramm auf.

Die Forderung nach Nachhaltigkeit in der Herstellung von persönlichen Hygieneartikeln zeigt sich in der Forderung nach biologischer Abbaubarkeit der einzelnen Komponenten wie Deckblatt, Rückenblatt, Speichersicht, Verteilerschicht. Zunehmend ist auch die Diskussion über Mikroplastik vorherrschend, d.h. Materialien, die aus synthetisch hergestellten Polymeren bestehen und durch "Abbau" nach ihrer Nutzung oder bereits durch Herstellung in Mikroplastik zerfallen. Als Mikroplastik bezeichnet man kleine Kunststoffteilchen mit einem Durchmesser unter 5 mm nach einer Definition der National Oceanic and Atmospheric Administration von 2008.[1]

Im Lichte des Vorstehenden, zeigt sich, dass bei Verwendung der bindemittel-verfestigten, cellulose-basierenden Deckblättern einerseits eine zu hohe Rücknässung vorliegt. Andererseits, und obwohl die zugrundeliegenden Fasern biologisch abbaubar sind, die Bindemittelpartikel nach Abbau der Fasermatrix als Mikroplastik verbleiben.

Bei der Verwendung von synthetischen Polymeren für die Herstellung von Deckblättern aus Filament- oder Stapelfaservliesstoffen ist die geringe biologische Abbaubarkeit nachteilig.

**Bestrebungen, Deckblätter aus biologisch abbaubaren Faserstoffen herzustellen, können dem Stand der Technik entnehmen.**

**So wird in der** US2015/0044926 A1 **die Verwendung von Mischungen aus hydrophilen mit hydrophoben, synthetisch hergestellten Cellulose-Fasern beschrieben. Zur Gewährleistung von hydrophilen Eigenschaften zur Flüssigkeitsaufnahme werden bis zu 75% Gewichtsprozent hydrophile Cellulose-Fasern eingesetzt und zusätzlich 1,0% Gewichtprozent Benetzungsmittel. Durch die Anwesenheit von hydrophilen Fasern ist zwar die Ansaugzeit gering, jedoch durch das Vorhandensein von dauerhaft hydrophilen Fasern im Vlies ist die Forderung nach geringer Rücknässung nicht gewährleistet. Gemäß der Tabelle 2 der** US2015/0044926 A1 **liegt die Rücknässung ("Rewet") bei 30%, dies wirkt sich negativ auf die Endkonsumenten-Wahrnehmung von Trockenheit im Hygieneprodukt aus.**

**Einen ähnlichen Ansatz verfolgt die** US2020/0138642A1**. Zur Gewährleistung der hydrophilen Eigenschaften wird für das Deckblatt ebenfalls eine Mischung von hydrophilen und hydrophoben natürlichen Cellulose-Fasern verwendet und ohne zusätzliche Beschichtung gearbeitet. Auch hier sind hydrophile Fasern dauerhaft in der Fasermischung im Einsatz, was die Forderungen nach geringer Rücknässung und die Endkonsumenten-Wahrnehmung zum Thema Trockenheit negativ beeinflusst.**

**Die** WO2015/041862A1 **beschreibt eine einseitig aufgebracht, dauerhaft hydrophile Beschichtung auf einem Vlies, das unter anderem auch aus cellulosischen Fasern bestehen kann. Diese Beschichtung ist mit dem Vlies dauerhaft verbunden, was zwar die Ansaugzeit positiv, aber die Rücknässung negativ beeinflusst. Des Weiteren zerfällt das Beschichtungsmittel nach Abbau der Fasermatrix in Mikroplastik-Partikel.**

Aufgabe war es daher, ein Deckblatt zu schaffen, welches die Nachteile **des Stands** der Technik vermeidet. Gelöst wird die Aufgabe anhand der Merkmale des Anspruches 1, vorteilhafte Ausgestaltungen sind in den Ansprüchen 2 - 5 genannt, um ein Deckblatt bereitzustellen, welches die biologische Abbaubarkeit gewährleistet, die Flüssigkeitsverteileigenschaften gewährleistet und dem Entstehen von Mikroplastik vorbeugt. Die nachstehenden Begriffe werden in der vorliegenden Erfindung genannt, wobei sich diese wie folgt definieren:
Die nachstehenden Messmethoden sind bei der EDANA mit Sitz in B-1160 Brussels, Belgium erhältlich:
- Rücknässung ist als "wet back" in der WSP 80.10 (09) beschrieben. Angabe in Gramm ("g")
- Ansaugzeit ist als "liquid strike through time" in NWSP 070.3 R1(19) beschrieben. Angabe in Sekunden ("s")
- Ermittlung der Sinkzeit von Fasern ist als "liquid absorbancy time" in der NWSP 010.1.R0(20), Methode 5.1 / 8.1 beschrieben. Angabe in Sekunden ("s")
- Flächengewicht als "mass per unit" in WSP 130.1, R0(20) , Angabe in g/m² Fasern mit hydrophoben Eigenschaften, kurz auch hydrophobe Faser: unter diesem Begriff sind Fasern zu verstehen, die wasserabweisende Eigenschaften aufweisen. Die wasserabweisenden Eigenschaften werden durch die Ermittlung der Sinkzeit von Faserproben beschrieben. Faserproben mit einer Sinkzeiten größer 600 Sekunden (danach wird im Allgemeinen der Test abgebrochen) werden als hydrophob bezeichnet, Faserproben mit einer Sinkzeit von kleiner 60 Sekunden werden als hydrophil bezeichnet. Faserproben mit einer Sinkzeit zwischen 60 und 600 Sekunden werden als semi-hydrophob bezeichnet. Sinkzeiten von Fasern können der Tabelle 2 entnommen werden.

Cellulosische Fasern: sind Fasern, die aus dem Grundmolekül Cellulose bestehen. Dies umfasst Faser aus natürlicher Cellulose wie Baumwolle oder Leinen und Faser aus regenerativ gewonnener Cellulose wie beispielsweise Viskose-Fasern. Alle fasern ist gemeinsam, das die Faser aus nachwachsenden Rohstoffen bestehen. Im Buch von Walther Loy "Chemiefaserstoffe - Aufbau - Eigenschaften - Verwendung", erschienen 1978 bei Schiele&Schön, Berlin ist der Aufbau von Naturfasern, im Speziellen Baumwolle, auf den Seiten 14-15 und Chemiefasern, im Speziellen Viskosefasern, auf den Seiten 28-34 erläutert.

Cellulosische Fasern aus natürlicher Cellulose sind beispielsweise Baumwollfaser. Sie bestehen aus Polymerketten des Makromoleküls Cellulose, wobei die Polymerketten einen durchschnittlichen Polymerisationsgrad von 2000-3000 aufweisen. Ungereinigte oder nur mechanisch gereinigte Baumwolle enthält auf der Oberfläche eine Wachsschicht aus natürlichen Substanzen wie Lipiden und/oder Polysacchariden. Darunter liegt eine weitere Schicht aus Pektinen, die eng mit der primären Zellwand der Fasern verbunden ist. Sowohl die Wachsschicht als auch die Pektinschicht haben hydrophobe Eigenschaften. Bei handelsüblicher Baumwolle, bspw für die Textilindustrie, werden die Baumwoll-Fasern zunächst mechanisch gereinigt um Schalenreste und anderen Fremdteile auszukämmen. Anschließend werden beide vorerwähnten hydrophoben Schichten durch beispielsweise Waschen zum Entfernen der Wachsschicht abgelöst und durch Peroxid-Bleichung der Pektinschicht aufgelöst um so die hydrophilen Eigenschaften der Baumwolle zu erhalten. Die Sinkzeit gebleichter Baumwolle liegt üblicherweise <1 Minute , die Sinkzeit rein mechanisch gereinigter Baumwolle weist üblicherweise eine Sinkzeit von >600Sekunden (=Abbruch des Tests) auf.

Cellulosische Fasern aus regenerativ gewonnener Cellulose sind beispielsweise Viskosefasern. Sie bestehen aus Polymerketten des Makromoleküls Cellulose, wobei die Polymerketten üblicherweise einen durchschnittlichen Polymerisationsgrad von 200-600 aufweisen. Handelsübliche Viskosefasern für die Textilindustrie haben üblicherweise auch hydrophile Eigenschaften. Die Sinkzeit beträgt üblicherweise <1 Minuten

Hydrophobe cellulosische Fasern: sind Fasern, die aus dem Grundmolekül Cellulose bestehen und entweder natürlich gebildete hydrophobe Substanzen beinhalten oder durch in Spinnmassen zugegebene Substanzen hydrophobe Eigenschaften aufweisen. Gattungsbildend ist auch die biologisch Abbaubarkeit gemäß einschlägiger Normen wie beispielsweise der DIN EN 12225:2000.

Hydrophobe Baumwolle ist eine rein mechanisch gereinigte, ungebleichte Baumwolle und weist aufgrund der natürlichen, trotz mechanischer Reinigung noch vorhandenen Wachs- und Pektinschicht hydrophobe Eigenschaften auf. Die Wachsschicht besteht aus Polysacchariden und Lipiden. Die Sinkzeit liegt dabei > 600 Sekunden (= Abbruch des Tests). Derartige Faser kann von WildWood Cotton Technologies, Greenwood, MS 38930 - USA unter der Bezeichnung von "TruCotton" bezogen werden.

Hydrophobe Viskosefaser: Durch die Zugabe von Hydrophobierungsmitteln in die Spinnmasse können Viskosefasern auch hydrophobe Eigenschaften verliehen werden. Beispielhaft ist hier die OLEA-Viskosefaser TYPE Z00774 der Firma Kelheim Fibers, Kelheim, Deutschland genannt mit einer Sinkzeit von >600 Sekunden (Abbruch des Tests).

Trockenverfahren: bezeichnet das Herstellverfahren für erfindungsgemäße Deckblätter. Erfindungsgemäße Deckblätter können nach einem im Buch "Vliesstoffe", erschienen im Verlag Wiley VCH, 2. Auflage, 2012 aufgezeigten Trockenverfahren hergestellt werden. Erfindungsgemäß bevorzugt, aber ohne darauf beschränkt zu sein, wird das Kardierverfahren unter Verwendung von Stapelfasern.

Wasserstrahlverfestigung: bezeichnet ein Verfestigungsverfahren für erfindungsgemäße Deckblätter. Die grundlegenden Techniken dazu werden im Buch "Vliesstoffe", erschienen im Verlag Wiley VCH, 2. Auflage, 2012, Seiten 340-359 beschrieben.

Oberflächliche, hydrophile Beschichtung: beschreibt ein Verfahren, bei dem auf ein textiles Flächengebilde wie einem Vliesstoff eine oberflächliche Auflage eines Hydrophilierungsmittel aufgebracht wird. Dies kann mittels üblicher kontaktloser Minimalauftragsverfahren, z.B. eines Rotations-Beschichtungssystem oder eines Besprühungsverfahren geschehen. erhältlich beispielsweise bei Weitmann & Konrad GmbH & Co. KG, 70771 Leinfelden-Echterdingen, Deutschland. Derartige Anlagen gewährleisten eine gleichmäßig über das Deckblatt verteilte Beschichtung, wobei diese Beschichtung im Bereich von 0,10 bis 0,45 Gewicht% in Bezug auf das Flächengewichts des Deckblatts liegen kann.

Hydrophilierungsmittel: ist ein mit wässrigen Salzlösungen, z.B. Urin oder anderen Körperflüssigkeiten leicht abwaschbares Mittel, dass die Benetzung und damit in der Folge die Flüssigkeitsannahme von Flächengebilden aus hydrophoben Faserstoffen verbessert. Beispielsweise kann dafür das Produkt Silastol R641 von Schill&Seilacher, 71032 Böblingen, Deutschland verwendet werden.

Ein erfindungsgemäßes Deckblatt besteht zu 100% aus hydrophoben Cellulose-Faser und weist eine leicht mit Körperflüssigkeiten abwaschbare, oberflächliche hydrophile Beschichtung auf. Ein derartiges Deckblatt kann, ohne darauf festgelegt zu sein, wie folgt hergestellt werden:
- Öffnen und Mischen der Faserballen hydrophober, cellulosischer Fasern
- Bildung eines unverfestigten Faserflores, bspw mittels kardieren
- Verfestigen des Faserflors mittels mechanischer Verfestigung durch beispielsweise Nadeln oder Wasserstrahlen
- Beschichten der Oberfläche des Vliesstoffes mit einem Hydrophilierungsmittel
- Trocknen des Vliesstoffes
- Wickeln / Schneiden

Die Faserballen werden geöffnet und mechanisch bearbeitet, sodass zusammenhängende Faserzöpfe oder Faserklumpen durch grobes Kämmen aufgelöst werden.

Die vorgeöffneten Fasern werden einem Vliesbildungsaggregat, bevorzugt einer Kardiereinheit vorgelegt, sodass mittels kardieren ein unverfestigter Faserflor im Flächengewichtsbereich von 20 bis 70g/m² bereitgestellt wird.

Dieser zunächst unverfestigte Faserflor wird dann einer nachfolgenden mechanischen Verfestigungseinheit vorgelegt. Zur Anwendung kann eine klassische Nadeleinheit mit Metallnadeln eingesetzt werden. Vorteilhaft ist in Hinblick auf die Verwendung des Vliesstoffes als Teil eines persönlichen Hygieneprodukts die Verfestigung via Wasserstrahlen bevorzugt.

Die hydrophoben Anteile der hydrophoben Cellulosefasern werden durch die mechanische Verfestigung nicht abgewaschen, sodass der so verfestigte Vliesstoff zunächst hydrophobe Eigenschaften, die in der Verwendung der hydrophoben cellulosischen Fasern begründet sind, aufweist.

Hydrophobierungsmittel in Viskose-Fasern, bspw den OLEA-Fasern, sind gut in der Matrix gebunden, daher werden die hydrophoben Eigenschaften auch nach Wasserstrahlverfestigung beibehalten.

Überraschend zeigte sich, dass bei Anwendung von Wasserstrahlverfestigung bei Baumwolle zwar die oberflächlich liegenden hydrophoben Lipide und Polysaccharide abgewaschen werden, die hydrophoben Pektine aber nicht. Allein das Vorhandensein der Pektine reicht aus, die hydrophoben Eigenschaften beizubehalten.

Der verfestigte, bei Verwendung von Wasserstrahlverfestigung noch nasser Flor wird dann im Anschluss an die Verfestigung einer Beschichtung zur oberflächlichen Hydrophilierung unterzogen. Vorteilhaft hat sich gezeigt, dass hier mit kontaktlosen Auftragssystemen, beispielsweise der Firma Weitmann & Konrad, Deutschland, eine genaue, gleichmäßig über den verfestigten Faserflor verteilte Dosierung der Beschichtungsmenge, auch im Bereich von 0,10 bis 0,50Gew% darstellbar ist.

Das Hydrophilierungsmittel wird dabei im Allgemeinen als wässrige Lösung aufgebracht. Die Behandlungslösung hat eine Konzentration von ca 2-10% Hydrophilierungsmittel-Anteil in der Dosierlösung. Im Rahmen der Ermittlung von Beschichtungsanteilen zur Gewährleistung der hydrophilen Eigenschaften wurden die Auflagemengen von Hydrophilierungsmittel im Bereich um ca 0,20 Gew%, ca 0,35 Gew% und ca 0,45 Gew% realisiert.

Die überschüssige Feuchtigkeit, die mittels der Beschichtung und, bei Wahl der Verfestigungsart Wasserstrahl, durch die Verfestigung in das Vlies eingetragen wird, wird mit einem nachfolgenden Trocknungsverfahren entfernt, sodass der so erfindungsgemäß hergestellt Vliesstoff eine Restfeuchte von 7 bis 13 Gewichts % und einen oberflächlichen liegenden Anteil Hydrophilierungsmittel von 0,20 bis 0,45 Gew% aufweist.

Das Flächengewicht eines so hergestellten Vliesstoffes liegt je nach Konstruktion des Hygieneartikels im Bereich von 20 bis 50 g/m², bevorzugt bei 25 bis 40g/m².

Die in Tabelle 1 genannten Vliesstoffe sind dabei wie folgt zusammengesetzt und hergestellt:

### Vergleichsbeispiel 1:

Faserzusammensetzung: 100% hydrophobe Viskosefaser, Olea TYPE Z00774 1,7dtex/40mm
Flächengewicht: 32g/m²
Herstellung: Kardiert / Wasserstrahlverfestigt
Beschichtungsanteil: ohne Beschichtung
Trocknungstemperatur: 110°C

### Vergleichsbeispiel 2: 100% Viskose

Faserzusammensetzung: 100% Lenzing Viskose 1,7dtex/40mm
Flächengewicht: 33g/m²
Herstellung: Kardiert / Wasserstrahlverfestigt
Beschichtungsanteil: ohne Beschichtung
Trocknungstemperatur: 110°C

### Vergleichsbeispiel 3:

Faserzusammensetzung: 100% mechanisch gereinigte Baumwoll-Faser TruCotton
Flächengewicht: 30g/m²
Herstellung: Kardiert / Wasserstrahlverfestigt
Beschichtungsanteil: ohne Beschichtung
Trocknungstemperatur: 110°C

### Vergleichsbeispiel 4:

Faserzusammensetzung: 100% Polypropylenfaser FiberVisions, DK, HyStrength 2,2dtex /40mm
Flächengewicht: 22g/m²
Herstellung: Kardiert / thermisch Kalander-verfestigt
Beschichtungsanteil: nicht anwendbar
Trocknungstemperatur: nicht anwendbar

### Erfindungsgemäßes Deckblatt 1:

Faserzusammensetzung: 100% mechanisch gereinigte Baumwolle TruCotton
Flächengewicht: 31g/m²
Herstellung: Kardiert / Wasserstrahlverfestigt
Beschichtungsanteil: 0,21Gew%
Trocknungstemperatur: 110°C

### Erfindungsgemäßes Deckblatt 2:

Faserzusammensetzung: 100% mechanisch gereinigte Baumwolle TruCotton
Flächengewicht: 31g/m²
Herstellung: Kardiert / Wasserstrahlverfestigt
Beschichtungsanteil: 0,30Gew%
Trocknungstemperatur: 110°C

### Erfindungsgemäßes Deckblatt 3

Faserzusammensetzung: 100% mechanisch gereinigte Baumwolle TruCotton
Flächengewicht: 31g/m²
Herstellung: Kardiert / Wasserstrahlverfestigt
Beschichtungsanteil: 0,45Gew%
Trocknungstemperatur: 110°C

### Erfindungsgemäßes Deckblatt 4

Faserzusammensetzung: 100% hydrophobe Viskosefaser, Olea TYPE Z00774 1,7dtex/40mm
Flächengewicht: 31g/m²
Herstellung: Kardiert / Wasserstrahlverfestigt
Beschichtungsanteil: 0,21 Gew%
Trocknungstemperatur: 110°C

### Erfindungsgemäßes Deckblatt 5

Faserzusammensetzung: 100% hydrophobe Viskosefaser, Olea TYPE Z00774 1,7dtex/40mm
Flächengewicht: 31g/m²
Herstellung: Kardiert / Wasserstrahlverfestigt
Beschichtungsanteil: 0,34 Gew%
Trocknungstemperatur: 110°C

### Erfindungsgemäßes Deckblatt 6

Faserzusammensetzung: 100% hydrophobe Viskosefaser, Olea TYPE Z00774 1,7dtex/40mm
Flächengewicht: 31g/m²
Herstellung: Kardiert / Wasserstrahlverfestigt
Beschichtungsanteil: 0,42 Gew%
Trocknungstemperatur: 110°C

Betrachtet man die Ansaugzeit in Tabelle 1, so erkennt man, dass ein unbeschichtetes Vlies aus hydrophoben cellulosischen Fasern eine Ansaugzeit im Bereich von 6 Sekunden bis 600 Sekunden aufweist.

Aus der Tabelle 1 kann man weiterhin erkennen, dass die hydrophile Beschichtung mit einer Menge von 0,30-0,35 Gew% eine Ansaugzeit von 3 Sekunden oder geringer gewährleistet.

Beschichtungsanteile größer als 0,45Gew% werden als nicht zielführend angesehen, da dann die Beschichtung die hydrophilen Eigenschaften die der hydrophoben Eigenschaften der cellulosischen Fasern überstrahlen. Siehe dazu die erfindungsgemäß ausgeführter Deckblätter 3 und 6, die Rücknässung liegt dicht an dem Grenzwert von 0,40g.

Sollte der Anteil der Beschichtung auf dem Deckblatt kleiner 0,20Gew% liegen, kommen die hydrophoben Anteile bereits zu Beginn zum Tragen, was bedeutet, dass die Ansaugzeit deutlich größer 3 Sekunden liegt und die Flüssigkeitsaufnahme entsprechend verschlechtert ist.

Ein erfindungsgemäß aufgebautes Deckblatt besteht einerseits aus biologisch abbaubaren, cellulosischen Faserstoffen auf Basis nachwachsenden Rohstoffen und weist andererseits die gewünschten hydrophilen / hydrophoben Eigenschaften für Deckblätter aus thermoplastischen Polymeren auf.

Ein erfindungsgemäß ausgeführtes Deckblatt ist daher für den Einsatz in persönlichen Hygieneprodukten geeignet und bietet eine gleichwertige Produktleistung im Vergleich zum Stands der Technik, ein erfindungsgemäß ausgeführtes Deckblatt ist aber biologisch abbaubar und bildet beim Abbau kein Mikroplastik der derzeitigen Definition.

**Tabelle 1:**

| **Produkt** | **Flächengewicht (g/m²)** | **Rücknässung (g)** | **Ansaugzeit (s)** |
|---|---|---|---|
| Vergleichsbeispiel 1 | 32 | 0,10 | >600 |
| Vergleichsbeispiel 2 | 33 | 8,22 | 1,5 |
| Vergleichsbeispiel 3 | 30 | 0,30 | 16,4 |
| Vergleichsbeispiel 4 | 22 | 0,14 | 2,7 |
| Erfindungsgemäßes Deckblatt 1 | 31 | 0,28 | 3,8 |
| Erfindungsgemäßes Deckblatt 2 | 31 | 0,30 | 3,3 |
| Erfindungsgemäßes Deckblatt 3 | 31 | 0,39 | 2,6 |
| Erfindungsgemäßes Deckblatt 4 | 31 | 0,12 | 4,4 |
| Erfindungsgemäßes Deckblatt 5 | 31 | 0,21 | 2,9 |
| Erfindungsgemäßes Deckblatt 6 | 31 | 0,38 | 2,9 |

**Tabelle 2:**

| Sinkzeit der Faserstoffe: | |
|---|---|
| **Faserstoff** | **Sinkzeit (s)** |
| Gebleichte Baumwolle | <10 |
| Mechanisch gereinigte Baumwolle | >600 |
| Handelsüblich Viskosefaser | <3 |
| Hydrophobe Viskosefasern | >600 |

## Patentansprüche

1. Deckblatt für persönliche Hygieneartikel
**dadurch gekennzeichnet, dass**
- das Deckblatt zu 100% aus hydrophoben cellulosischen Fasern besteht, **deren Sinkzeit gemessen nach Prüfmethode NWSP 010.1R0(20), 5.118.1 größer 600 Sekunden beträgt,**
- das Deckblatt mechanisch verfestigt ist und
- das Deckblatt eine oberflächliche, hydrophile Beschichtung enthält

2. Deckblatt nach Anspruch 1,
**dadurch gekennzeichnet,**
- **dass** die hydrophobe cellulosische Faser eine mechanisch gereinigte, ungebleichte Baumwolle ist

3. Deckblatt nach Anspruch 1,
**dadurch gekennzeichnet,**
- **dass** die hydrophobe cellulosische Faser hydrophobe Viskosefaser ist

4. Deckblatt nach Anspruch 1,
**dadurch gekennzeichnet,**
- **dass** das Deckblatt mittels Wasserstrahlverfestigung verfestigt ist.

5. Deckblatt nach Anspruch 1,
**dadurch gekennzeichnet,**
- **dass** die **Auflagemenge der oberflächlichen, hydrophilen** Beschichtung nicht mehr als 0,45 Gewichtsprozent beträgt und
- **die oberflächliche, hydrophile Beschichtung abwaschbar ist.**

## Claims

1. A cover sheet for personal hygiene articles
**characterized in that**
- the cover sheet consists of 100% hydrophobic cellulosic fibers, the sink time thereof being greater than 600 seconds, when measured by test method NWSP 010.1R0(20), 5.1/8.1,
- the cover sheet is mechanically consolidated, and
- the cover sheet contains a hydrophilic surface coating.

2. The cover sheet according to claim 1,
**characterized in that**
- the hydrophobic cellulosic fiber is a mechanically cleaned, unbleached cotton.

3. The cover sheet according to claim 1,
**characterized in that**
- the hydrophobic cellulosic fiber is hydrophobic viscose fiber.

4. The cover sheet according to claim 1,
**characterized in that**
- the cover sheet is consolidated by means of hydro-entanglement.

5. The cover sheet according to claim 1,
**characterized in that**
- the coating amount of the hydrophilic surface coating is not more than 0.45% by weight, and
- the hydrophilic surface coating is washable.

## Revendications

1. Feuille de dessus pour article d'hygiène personnelle
**caractérisée en ce que**
- la feuille de dessus est composée à 100 % de fibres cellulosiques hydrophobes dont le temps de descente, mesuré selon la méthode d'essai NWSP 010.1R0 (20), 5.1/8.1, est supérieur à 600 secondes,
- la feuille de dessus est solidifiée mécaniquement, et
- la feuille de dessus contient un revêtement hydrophile superficiel.

2. Feuille de dessus selon la revendication 1,
**caractérisée en ce que**
- la fibre cellulosique hydrophobe est un coton non blanchi nettoyé mécaniquement.

3. Feuille de dessus selon la revendication 1,
**caractérisée en ce que**
- la fibre cellulosique hydrophobe est une fibre de viscose hydrophobe.

4. Feuille de dessus selon la revendication 1,
**caractérisée en ce que**
- la feuille de dessus est solidifiée au moyen d'un jet d'eau.

5. Feuille de dessus selon la revendication 1,
**caractérisée en ce que**
- la quantité d'application du revêtement hydrophile superficiel ne dépasse pas 0,45 % en poids, et
- le revêtement hydrophile superficiel est lavable.
